# EUROPÄISCHE PATENTSCHRIFT

(11) **EP 3 932 389 B1**
(45) Veröffentlichungstag und Bekanntmachung des Hinweises auf die Patenterteilung: **08.04.2026**
(21) Anmeldenummer: 20183179.9
(22) Anmeldetag: 30.06.2020
(51) Int. Cl.: A61K 47/38, A61K 47/36, A61K 47/32, A61K 47/10, A61K 47/02, A61K 38/44, A61K 9/10, A61K 9/08, A61K 9/00, A61P 31/14, A61P 11/00, A61K 31/717, A61K 31/77, A61K 31/728

(54) **ZUSAMMENSETZUNG ZUR BEHANDLUNG UND PRÄVENTION VON MINDESTENS EINEM SYMPTOM EINER MIT COVID-19 ASSOZIIERTEN SEKUNDÄREN ERKRANKUNG BEIM MENSCHEN**
COMPOSITION FOR THE TREATMENT AND PREVENTION OF AT LEAST ONE SYMPTOM OF A SECONDARY DISEASE ASSOCIATED WITH COVID-19 IN HUMANS
COMPOSITION DE TRAITEMENT ET DE PRÉVENTION D'AU MOINS UN SYMPTÔME D'UNE MALADIE SECONDAIRE CHEZ DES PERSONNES ASSOCIÉES AU COVID-19

(43) Veröffentlichungstag der Anmeldung: 05.01.2022
(73) Patentinhaber: Schmidt, Werner, 51580 Reichshof (DE)
(72) Erfinder: Schmidt, Werner, 51580 Reichshof (DE)
(74) Vertreter: Fuchs Patentanwälte Partnerschaft mbB

(56) Entgegenhaltungen:
- DE-A1- 102016 125 378
- CHU DEREK K ET AL: "Physical distancing, face masks, and eye protection to prevent person-to-person transmission of SARS-CoV-2 and COVID-19: a systematic review and meta-analysis", THE LANCET, ELSEVIER, AMSTERDAM, NL, vol. 395, no. 10242, 1 June 2020 (2020-06-01), pages 1973 - 1987, XP086202886, ISSN: 0140-6736, [retrieved on 20200601], DOI: 10.1016/S0140-6736(20)31142-9
- RKI: "Gibt es antivirale Substanzen, die zur Behandlung von COVID-19 zur Verf�gung stehen?", 19 June 2020 (2020-06-19), pages 1 - 5, XP055956790, Retrieved from the Internet <URL:https://edoc.rki.de/bitstream/handle/176904/6852/COVRIIN_Antivirale%20Therapie_20200618.pdf?sequence=1&isAllowed=y> [retrieved on 20220901], DOI: 10.25646/6953
- WEIBIAO ZENG ET AL.: "Association of Daily Wear of Eyeglasses With Susceptibilityto Coronavirus Disease 2019 Infection", JAMA OPHTHALMOLOGY, vol. 138, no. 11, November 2020 (2020-11-01), pages 1196 - 1198
- SHETTY ROHIT ET AL: "Potential ocular and systemic COVID-19 prophylaxis approaches for healthcare professionals", INDIAN JOURNAL OF OPHTHALMOLOGY, vol. 68, no. 7, 25 June 2020 (2020-06-25), IN, pages 1349, XP093241608, ISSN: 0301-4738, Retrieved from the Internet <URL:https://pmc.ncbi.nlm.nih.gov/articles/PMC7574070/pdf/IJO-68-1349.pdf> DOI: 10.4103/ijo.IJO_1589_20

## Beschreibung

### Einleitung

Die vorliegende Erfindung betrifft eine sterile, wässrige Zusammensetzung zur Anwendung in einem Verfahren für die Behandlung und Prävention von mindestens einem Symptom einer mit COVID-19 assoziierten sekundären Erkrankung beim Menschen, umfassend das Einbringen der Zusammensetzung in das Auge.

Die Erkrankung COVID-19 ist eine Infektionskrankheit, zu der es infolge einer Infektion mit dem neuartigen Coronavirus SARS-CoV-2 kommen kann. Die primär die Atemwege befallende Erkrankung wurde erstmals Ende des Jahres 2019 in Wuhan beschrieben, entwickelte sich im Januar 2020 in der Volksrepublik China zur Epidemie und breitete sich dann weltweit zur COVID-19-Pandemie aus.

Die Ansteckung der viralen Infektion erfolgt in der Regel durch Tröpfchenübertragung. Unter bestimmten Umständen ist auch eine Übertragung über Aerosole möglich. Die Krankheitsverläufe sind unspezifisch, vielfältig und variieren stark.

Als ein Grund für die starke Variation der Krankheitsverläufe wird vermutet, dass durch die Schwächung des Immunsystems durch das SARS-CoV-2-Virus als Primärinfekt, sekundäre, meist bakterielle Infektionen für eine Verschlechterung des Krankheitsverlaufs sorgen. Diese Hypothese würde auch erklären, warum manche Patienten keine oder nur milde Krankheitsverläufe zeigen, während andere Patienten lebendbedrohlich erkranken.

Die Inkubationszeit einer SARS-CoV-2-Infektion beträgt durchschnittlich fünf bis sechs Tage; zwischen Ansteckung und dem Auftreten erster Symptome können aber auch bis zu zwei Wochen vergehen. Vereinzelt treten erste Symptome schon innerhalb von 24 Stunden nach Ansteckung mit SARS-CoV-2 auf. Am häufigsten sind Fieber, trockener Husten und Müdigkeit. Weniger häufig sind Schmerzen, eine verstopfte Nase, Kopfschmerzen, Halsschmerzen, Durchfall, Geschmacks- oder Geruchsverlust oder Hautausschlag oder Verfärbung von Fingern oder Zehen.

Es wurde nachgewiesen, dass auch Infizierte ohne Symptome trotzdem potenzielle Überträger des Coronavirus sein können.

Obwohl COVID-19 zurzeit Gegenstand intensiver Forschung ist, gibt es, außer den von der WHO empfohlenen Verhaltensanpassungen, bislang keine ausreichenden Mittel, die effizient zur Prävention und Behandlung von mindestens einem Symptom einer mit COVID-19 assoziierten sekundären Erkrankung geeignet sind.

DE 10 2016 125378 A1 offenbart Augentropfen zur Prävention von Erkältungskrankheiten. Eine Behandlung von Covid-19 oder anderen SARS-vermittelten Virusinfektionen ist nicht offenbart.

CHU DEREK K ET AL: "Physical distancing, face masks, and eye protection to prevent personto-person transmission of SARS-CoV-2 and COVID-19: a systematic review and meta-analysis", THE LANCET, ELSEVIER, AMSTERDAM, NL, Bd. 395, Nr. 10242, 1. Juni 2020 (2020-06-01), Seiten 1973-1987, XP086202886, ISSN: 0140-6736, DOI: 10.1016/S0140-6736(20)31142-9; offenbart eine Meta-Analyse nach der das Tragen von Augenprotektoren (Brillen oder Gesichtsschilde) möglicherweise die Infektion mit SARS-CoV-2 reduziert.

RKI: "Gibt es antivirale Substanzen, die zur Behandlung von COVID-19 zur Verfügung stehen?", 19. Juni 2020 (2020-06-19), Seiten 1-5, XP055956790, DOI: 10.25646/6953 URL:https://edoc.rki.de/bitstream/handle/176904/6852/ offenbart die diskutierten und verwendeten Behandlungsmethoden zur Behandlung von Covid-19 assoziierten Symptomen. Augentropfen werden nicht diskutiert.

Shetty Rohit ET AL: "Potential ocular and systemic COVID-19 prophylaxis approaches for healthcare professionals", Indian Journal of Ophthalmology, Bd. 68, Nr. 7, 25. Juni 2020 (2020-06-25), Seite 1349, XP093241608, IN, ISSN: 0301-4738, DOI: 10.4103/ijo.IJO_1589 20; offenbaren verschiedene Ansätze zur Behandlung von Covid-19 mittels Augentropfen, wobei diese Augentropfen aber mindestens einen Wirkstoff enthalten, dem eine Covid-19-inhibierende Wirkung zugeschrieben wird.

### Aufgabe der Erfindung

Die vorliegende Erfindung hat zur Aufgabe ein effizientes Mittel zur Anwendung in einem Verfahren für die Behandlung und Prävention von mindestens einem Symptom einer mit COVID-19 assoziierten sekundären Erkrankung beim Menschen bereitzustellen. Die Erfindung soll eine wirksame Behandlung und Prävention von mindestens einem Symptom einer mit COVID-19 assoziierten sekundären Erkrankung bereits in einem frühen Stadium, also zu Beginn der Infektion ermöglichen und wenn die Infektion ausgebrochen ist, soll sie den Ablauf abmildern und die Krankheitsdauer stark verringern. Das Mittel soll außerdem kostengünstig und einfach applizierbar sein.

Diese Aufgabe wird durch das erfindungsgemäße Mittel gelöst.

### Beschreibung der Erfindung

Die Erfindung betrifft eine sterile, wässrige Zusammensetzung zur Anwendung in der Behandlung oder Prävention von mindestens einem Symptom einer mit COVID-19 assoziierten sekundären Erkrankung bei einem Menschen, ausgewählt aus Fieber, trockenem Husten, Müdigkeit, Gliederschmerzen, Halsschmerzen, Durchfall, Kopfschmerzen, Verlust des Geschmacks- oder Geruchssinns, Verfärbungen an Fingern oder Zehen, Hautausschlag, Atembeschwerden oder Kurzatmigkeit, Schmerzen oder Druckgefühl im Brustbereich, Verlust der Sprach- oder Bewegungsfähigkeit, Muskelschmerzen, Rückenschmerzen, Brustschmerzen im Sinne einer Pleuritis, Verminderung der Anzahl der gesamten weißen Blutzellen, Verminderung der Lymphozyten-Anzahl, einer Erhöhung laborchemischer Entzündungsparameter (wie CRP und BSG), Schnupfen, Übelkeit und einer Lungenentzündung (Pneumonie),
- wobei der Mensch dadurch definiert wird, dass bei ihm eine SARS-CoV-2-Infektion mittels quantitativer Polymerase-Kettenreaktion (PCR) nachgewiesen wurde,
- umfassend das Einbringen der Zusammensetzung in das Auge,
- wobei die Zusammensetzung während der Wachphase des Menschen und mehrmals mit einem zeitlichen Abstand von höchstens 2 h in das Auge eingebracht wird;
- wobei die Zusammensetzung eine sterile, wässrige Zusammensetzung einer hypotonischen Lösung von NaCl und KCI (275 mOsm/l) ist, weiterhin umfassend 0,24 % Verdickungsmittel, Phosphatpuffer (Di-Natriumphosphat-Dodecahydrat/Na-Dihydrogenphosphat), pH-Wert 7,2, und optional zusätzlich 0,5 Gew.-% Na₂EDTA,
- wobei die Zusammensetzung frei von pharmazeutischen Wirkstoffen, insbesondere frei von abschwellenden Wirkstoffen, Antihistaminika, Antibiotika, Ephedrin und/oder Pseudoephedrin ist.

Überraschenderweise hat der Erfinder der vorliegenden Erfindung festgestellt, dass das Einbringen der erfindungsgemäßen Zusammensetzung in das Auge eine effiziente Behandlung und Prävention von mindestens einem Symptom einer mit COVID-19 assoziierten sekundären Erkrankung beim Menschen ermöglicht.

Hierbei bezieht sich der Begriff "SARS-CoV-2" (Abk. für englisch: *severe acute respiratory syndrome coronavirus*, deutsch: Schweres-akutes-Atemwegssyndrom-Coronavirus 2), auf das Virus gehörend zur Familie der Coronaviren. Eine Infektion mit diesem Virus verursacht die neue Atemwegserkrankung COVID-19.

Hierbei bezieht sich der Begriff *"mit COVID-19 assoziierte sekundäre Erkrankung"* auf die Behandlung oder Prävention von mindestens einem Symptom einer mit COVID-19 assoziierten sekundären Erkrankung bei einem Menschen, ausgewählt aus Fieber, trockenem Husten, Müdigkeit, Gliederschmerzen, Halsschmerzen, Durchfall, Kopfschmerzen, Verlust des Geschmacks- oder Geruchssinns, Verfärbungen an Fingern oder Zehen, Hautausschlag, Atembeschwerden oder Kurzatmigkeit, Schmerzen oder Druckgefühl im Brustbereich, Verlust der Sprach- oder Bewegungsfähigkeit, Muskelschmerzen, Rückenschmerzen, Brustschmerzen im Sinne einer Pleuritis, Verminderung der Anzahl der gesamten weißen Blutzellen, Verminderung der Lymphozyten-Anzahl, einer Erhöhung laborchemischer Entzündungsparameter (wie CRP und BSG), Schnupfen, Übelkeit und einer Lungenentzündung (Pneumonie).

Wobei die Sekundärinfektion, meist ausgelöst durch Bakterien, hervorgerufen wird. In diesem Sinne ist z.B. eine Lungenentzündung, die durch *Streptococcus pneumoniae* hervorgerufen wird, eine *"mit SARS-CoV-2 assoziierte sekundären Erkrankung COVID-19"* sofern sie als Folge- bzw. Sekundärinfektion während oder nach einer akuten SARS-CoV-2-Infektion auftritt.

Weitere bekannte Ursachen für Sekundärinfektionen im Sinne der Erfindung sind häufig multiresistente, gram-negative Bakterien, wie z.B. *Acinetobacter baumannii* oder *Klebsiella pneumoniae.* Dies ist insbesondere gefährlich, da gegen gramnegative Bakterien die Entwicklung neuer Antibiotika besonders schwierig ist. Die Substanz Cefiderocol sei eines der ganz wenigen neuen Antibiotika, die hier eine gewisse Wirksamkeit zeigen.

Daher eignet sich die erfindungsgemäße Zusammensetzung insbesondere zur Prävention und Behandlung von Sekundärinfektionen mit Bakterien ausgewählt aus *Staphylococcus aureus, Streptococcus pneumoniae, Koagulase-negative Staphylokokken, Enterococcus faecium, Enterococcus faecalis, Escherichia coli, Klebsiella pneumoniae, Enterobacter cloacae, Serratia marcescens, Citrobacter spp., Pseudomonas aeruginosa, Acinetobacter baumannii* und *Stenotrophomonas maltophilia.*

In weiteren Ausführungsformen andere Bakterien, die sich insbesondere im Speichel und Tröpfchen finden lassen, wie z.B. Meningokokken, Streptokokken, *Bordetella pertussis* und Pneumokokken.

In weiteren Ausführungsformen eignet sich erfindungsgemäße Zusammensetzung zur Prävention und Behandlung von Sekundärinfektionen mit Pilzen (Mykosen), wie z.B. *Candida spp., Aspergillus spp., Mucorales spp.* und *Fusarium spp.*; oder Viren, wie z.B. Herpes-simplex-Viren Typ I, Epstein-Barr-Virus, Influenza-Viren, Morbilliviren, Paramyxoviren, Mumps, Rubellavirus, Varizella-Zoster-Viren, Erkältungs-Viren (wie z.B. harmlose Coronaviren wie z.B. HCoV-HKU1, HCoV-OC43, HCoV-NL63 und HCoV-229E, Rhinoviren, etc.).

Es ist jedenfalls erstaunlich und höchst unerwartet, dass das Einbringen der erfindungsgemä-ßen Zusammensetzung in das Auge eine effiziente Behandlung und Prävention von mindestens einem Symptom einer mit COVID-19 assoziierten sekundären Erkrankung beim Menschen ausgewählt aus Fieber, trockenem Husten, Müdigkeit, Gliederschmerzen, Halsschmerzen, Durchfall, Kopfschmerzen, Verlust des Geschmacks- oder Geruchssinns, Verfärbungen an Fingern oder Zehen, Hautausschlag, Atembeschwerden oder Kurzatmigkeit, Schmerzen oder Druckgefühl im Brustbereich, Verlust der Sprach- oder Bewegungsfähigkeit, Muskelschmerzen, Rückenschmerzen, Brustschmerzen im Sinne einer Pleuritis, Verminderung der Anzahl der gesamten weißen Blutzellen, Verminderung der Lymphozyten-Anzahl, einer Erhöhung laborchemischer Entzündungsparameter (wie CRP und BSG), Schnupfen, Übelkeit und einer Lungenentzündung (Pneumonie) ermöglicht. Bekannte Anwendungen von wässrigen Zusammensetzungen am Auge dienen bekanntlich lediglich zur lokalen Behandlung von Erkrankungen des Auges. Die Anwendung einer Zusammensetzung am Auge zur Behandlung und Prävention von Symptomen einer mit COVID-19 assoziierten sekundären Erkrankung, die bekanntermaßen nicht am Auge auftreten, sondern in der Regel eine entsprechende Symptomatik in Hals, Nase oder Lunge der betroffenen Personen verursachen, ist bis dato unbekannt.

Es ist daher nochmals hervorzuheben, dass die Behandlung von Augenerkrankungen oder Augensymptomen, wie z.B. Bindehautentzündungen nicht von dem vorliegenden erfindungsgemä-ßen Einsatz der Zusammensetzung umfasst sein sollen.

Ohne an eine bestimmte Wirkungsweise gebunden zu sein, ist es denkbar, dass die erfindungsgemäße Zusammensetzung durch Anwendung am Auge über den Tränenkanal oder andere Kanäle zum oberen Nasenraum gelangen kann und dort ihre Wirkung entfalten kann. Vorzugsweise kann so die Luftzufuhr zum oberen Nasenraum aufrechterhalten werden. Eine verbesserte Luftzufuhr und/oder Befeuchtung der Schleimhäute verhindert oder verzögert das Einnisten der Erreger und/oder kann zum Absterben der dort vorhandenen Erreger beitragen. Eine Ausbreitung der Infektion beispielsweise in tiefe Bereiche des Respirationssystems kann so vorteilhafterweise vorgebeugt, abgemildert, eingedämmt oder verhindert werden. Folgeerkrankungen wie zum Beispiel eine Lungenentzündung werden vorteilhafterweise weniger wahrscheinlich.

Ohne an eine besondere Wirkungsweise gebunden zu sein, wird vermutet, dass in einer Ausführungsform die erfindungsgemäße Zusammensetzung die sekundäre Infektiosität nach einer SARS-CoV-2-Infektion herabsetzt, indem sie das Eindringen und Festsetzen des sekundären Erregers erschwert. In einer bevorzugten Ausführungsform wirkt die Zusammensatzung durch die Verringerung und/oder Verhinderung der gefährlichen Folgewirkungen der viralen Infektion, welche durch Bakterien hervorgerufen werden. Vor allem eine lebensgefährliche Lungenentzündung kann in vielen Fällen durch die erfindungsgemäße Zusammensetzung vermieden werden.

Die WHO empfiehlt, neben dem Einhalten von Abstandsregeln und Hygienemaßnamen, die Verwendung von Gesichtsmasken zur Verringerung des Infektionsrisikos. Hierbei ergeben sich aber gleich mehrere Probleme:
Einerseits ergibt sich das Problem, dass das regelmäßige Tragen der Masken zu einem feuchtwarmen Milieu führt, welches das Wachstum von Viren, Bakterien und Pilzen fördert. Da die Masken direkt vor Mund und Nase getragen werden, können, insbesondere bei falscher Anwendung, diese potentiell gefährlichen Erreger inhaliert werden.

Die durch die Masken verursachte schlechte Atemluft sorgt paradoxerweise außerdem für trockene Schleimhäute. Ohne ausreichenden Feuchtigkeitsfilm können sich Krankheitserreger wie Viren, Pilze und Bakterien noch ungehinderter auf die ausgetrockneten Schleimhäute setzen und sich in der Nase, sowie im Mund- und Rachenraum vermehren.

Dies gilt insbesondere für Maskentypen, bei denen der Atemstrom so umgeleitet wird, dass ein erheblicher Teil des Atems in den Lücken neben den Nasenflügeln an den Augen vorbei ausströmt, was zur Steigerung der Empfindlichkeit der Augen nicht nur gegenüber einer potentiellen SARS-CoV-2-Infektion, sondern auch gegenüber Sekundärinfektionen führt.

Daher kann in einer weiteren Ausführungsform der Einsatz der erfindungsgemäßen Zusammensetzung auch bei der Prävention einer COVID-19 assoziierten sekundären Erkrankung helfen, indem die Anfälligkeit der Augen und Tränenkanäle für die Erreger beim Tragen von Gesichtsmasken verringert wird.

Der präventive Einsatz der erfindungsgemäßen Zusammensetzung insbesondere beim Tragen von Gesichtsmasken ist daher eine weitere bevorzugte Ausführungsform der vorliegenden Erfindung.

### Zusammensetzung

Die vorliegende Erfindung stellt eine sterile, wässrige Zusammensetzung zur Anwendung in einem Verfahren für die Behandlung und Prävention von mindestens einem Symptom einer mit COVID-19 assoziierten sekundären Erkrankung beim Menschen bereit. Das Verfahren umfasst das Einbringen der Zusammensetzung in das Auge.

Die erfindungsgemäße Zusammensetzung ist eine sterile, wässrige Zusammensetzung einer hypotonischen Lösung von NaCl und KCI (275 mOsm/l) ist, weiterhin umfassend 0,24 % Verdickungsmittel, Phosphatpuffer (Di-Natriumphosphat-Dodecahydrat/Na-Dihydrogenphosphat), pH-Wert 7,2, und optional zusätzlich 0,5 Gew.-% Na₂EDTA.

Durch entsprechendes Einstellen der Osmolarität werden gute Verträglichkeiten am Auge erreicht.

Eine gute Verträglichkeit bedeutet, dass beim Einbringen der hier beschriebenen Zusammensetzung im Regelfall keine wesentlichen Reizungen des Auges oder Schmerzen am Auge auftreten.

Für eine gute Verträglichkeit am Auge spielt außerdem der pH-Wert der Zusammensetzung eine Rolle. Zusammensetzungen mit pH-Werten im Bereich von 6,6 bis 10,5 gelten im Auge als physiologisch verträglich. Zusammensetzungen mit pH-Werten über 10,5 oder unter 6,6 werden in der Regel im Auge nicht gut vertragen und können zu Schmerzen oder Irritationen oder beidem führen.

Die Zusammensetzung umfasst weiterhin NaCl und KCI. Dieses kann vorteilhafterweise zur Einstellung der oben genannten Osmolarität dienen.

Die Zusammensetzung umfasst Natriumchlorid und Kaliumchlorid.KCl und insbesondere Natriumchlorid, sind vorteilhaft, da es sich um ausgesprochen gut verträgliche Salze handelt, die für die Einstellung der Osmolarität gut geeignet sind.

Vorzugsweise umfasst die Zusammensetzung das anorganische Salz in einer Konzentration von 0,7 bis 1,4 Gew.-% bezogen auf das Gesamtgewicht der Zusammensetzung. Mehr bevorzugt weist die Zusammensetzung das anorganische Salz in einer Konzentration von 0,7 bis 1,2 Gew.-% und besonders bevorzugt von 0,8 bis 1,0 Gew.-% bezogen auf das Gesamtgewicht der Zusammensetzung auf. Am meisten bevorzugt weist die Zusammensetzung Natriumchlorid in einer Konzentration von 0,7 bis 1,2 Gew.-% bezogen auf das Gesamtgewicht der Zusammensetzung auf.

Die oben genannten Salzkonzentrationen sind vorteilhaft, weil sie eine dem osmotischen Druck des Auges angepasste Osmolarität besitzen. Eine angepasste Osmolarität kann vorteilhafterweise zur Minimierung von Schmerz- und Reizerscheinungen am Auge beitragen, die durch Einbringen der Zusammensetzung verursacht sein können.

Die Zusammensetzung umfasst weiterhin vorzugsweise ein Verdickungsmittel. Besonders bevorzugt ist das Verdickungsmittel ausgewählt aus Polyethylenglykol (PEG); Propylenglykol; Polyvinylpyrrolidon; Polyvinylalkohol; Cellulose; Cellulosederivaten, wie beispielsweise Carboxymethylcellulose und Hydroxypropylmethylcellulose; Xanthan; Carbomere; Derivate des Guar-Gummis, wie Hydroxypropyl-Guar; Hyaluronsäure und deren Derivaten, sowie Mischungen daraus. Am meisten bevorzugt sind Verdickungsmittel ausgewählt aus der Gruppe Hyaluronsäure, PEG und Hydroxypropylmethylcellulose. Der Zusatz von Verdickungsmittel in der Zusammensetzung ist vorteilhaft, da so die Viskosität der Zusammensetzung beeinflusst werden kann. Vorteilhafterweise bewirkt eine Erhöhung der Viskosität, dass die Kontaktzeit zwischen Zusammensetzung und Auge verlängert wird.

Ein weiterer Vorteil des Zusatzes von Verdickungsmitteln ist, dass sie Wasser binden und eine lubrifizierende Wirkung haben. Durch den Zusatz von Verdickungsmitteln kann die Zusammensetzung die Hornhaut besonders gut benetzen und auf der Gewebeoberfläche länger haften als ohne den Zusatz von Verdickungsmitteln. Insbesondere kann beispielsweise eine verlängerte Haftung an der schleimhaltigen Phase des Tränenfilms erreicht werden. Dieser Effekt ist als Mukoadhäsion bekannt. Somit ergibt sich zusätzlich eine längere Kontaktzeit am Auge.

Vorzugsweise ist das Verdickungsmittel in der Zusammensetzung in einer Menge von 0,1 bis 10 Gew.-% bezogen auf das Gesamtgewicht der Zusammensetzung enthalten. Mehr bevorzugt ist das Verdickungsmittel in der Zusammensetzung in einer Menge von 0,15 bis 5 Gew.-% bezogen auf das Gesamtgewicht der Zusammensetzung enthalten und am meisten bevorzugt enthält die Zusammensetzung das Verdickungsmittel in einer Menge von 0,2 bis 1 Gew.-% bezogen auf das Gesamtgewicht der Zusammensetzung.

Die Zusammensetzung ist frei von pharmazeutischen Wirkstoffen, insbesondere frei von abschwellenden Wirkstoffen wie Antihistaminika; Antibiotika; Schmerzmitteln, wie Paracetamol, Ibuprofen und/oder Acetylsalicylsäure und deren Derivaten; Ephedrin, Pseudoephedrin und/oder anderen. In einer Ausführungsform ist die Zusammensetzung frei von Hydroxychloroquin, Interferonen und/oder Trehalose.

Besonders bevorzug ist die Zusammensetzung frei von einem oder mehreren der folgenden

Wirkstoffe: Acetylcystein; Ambroxol; Andornkraut; Angelikawurzel; Anisöl; Benzocain;Bittersüßstängel; Bromhexin; Cajeputöl; Campher; Carbocistein; Cineol; Clenbuterol; Clobutinol; Codein; Coffein; Chlorphenamin; Dextromethorphan; Dihydrocodein; Dimetinden; Diphenylpyralin; Doxycyclin; Doxylamin; Dropropizin; Efeublätter; Eibisch; Enzianwurzel; Ephedrin; Eukalyptusöl; Eisenkraut; Eukalyptusblätter; Färberhülsenwurzel; Fichtennadelöl; Guaifenesin; Holunderblüten; Hydrocodon; Isländisch Moos; Kapuzinerkressekraut; Kiefernnadelöl; Koniferenöl; Lebensbaumspitzen; Levomenthol; Lindenblüten; Meerrettichwurzel; Metamfepramon; Menthol; Myrtenöl; Myrtol; Naphazolin; Noscapin; Oxymetazolin; Oxytetracyclin; Paracetamol; Pelargoniumwurzel; Pentoxyverin; Pfefferminzöl; Pheniramin; Phenylephrin; Pseudoephedrin; Sauerampferkraut; Schlüsselblumen; Schlüsselblumenblüten mit Kelch; Sonnenhut; Spitzwegerichkraut; Süßorangenöl; Terpentinöl; Thymian; Thymiankraut; Thymianöl; Thymol; Tramazolin; Triprolidin; Tyrothricin; Vitamin C; Weidenrinde; Xylometazolin; Zitronenöl.

In einer anderen Ausführungsform enthält die Zusammensetzung zusätzlich einen der zuvor genannten Wirkstoffe, um einen Synergie-Effekt zwischen der Wirkung der Zusammensetzung und der des Wirkstoffes zu erhalten.

Weiterhin umfasst die Zusammensetzung vorzugsweise ein Puffersystem. Der Zusatz eines Puffersystems ist vorteilhaft, da sich so der oben beschriebene pH-Wert der Zusammensetzung beim Vermischen mit Tränenflüssigkeit beim Einbringen in das Auge nicht oder kaum ändert. Besonders bevorzugt umfasst die Zusammensetzung ein Phosphat- puffersystem. Phosphatpuffersysteme sind in der Regel physiologisch gut verträglich und somit vorteilhaft.

Vorzugsweise umfasst die Zusammensetzung weitere Hilfsstoffe. Bevorzugte Hilfsstoffe sind beispielsweise Geliermittel, Konservierungsmittel, Komplexbildner und Enzyme.

Vorzugsweise kann die Zusammensetzung einen Komplexbildner enthalten. Geeignete Komplexbildner sind beispielsweise EDTA, beispielsweise in Form des Di-Natrium-Salzes, oder Citrat. Die Zusammensetzung kann auch mehrere Komplexbildner enthalten. Komplexbildner sind vorteilhaft, da sie zweiwertige Ionen binden können. So kann beispielsweise die Präzipitation von Kalziumphosphat, Magnesiumphosphaten oder anderen zweiwertigen Phosphaten oder Salzen aus der Zusammensetzung verhindert werden. Eine Kalzifizierung der Hornhaut (beispielsweise durch Bildung schwer löslicher Kalziumphosphat-Verbindungen) kann dadurch vorteilhafterweise unterbunden werden.

Vorzugsweise liegt der oder liegen die Komplexbildner in einer Gesamtkonzentration von 0,001 bis 1 Gew.-% in der Zusammensetzung vor. Mehr bevorzugt liegt der oder liegen die Komplexbildner in einer Gesamtkonzentration von 0,005 bis 0,5 Gew.-% in der Zusammensetzung vor.

Bevorzugte Geliermittel umfassen die oben genannten Verdickungsmittel und können alternativ oder zusätzlich eine oder mehrere der folgenden Substanzen umfassen: Alginate, Agar-Agar, Carrageen, Furcellaran, Gellan; Pektin und Pektin-Derivate; Gummi arabicum; Methylcellulose; Ethylcellulose; Methylethylcellulose; Hydroxypropylcellulose; Gelatine; modifizierte Stärke. Der Zusatz von Geliermittel kann vorteilhaft sein, um die Viskosität zu erhöhen. Somit können die Geliermittel vorteilhafterweise dazu dienen, die Zusammensetzung als Salbe oder Gel herzurichten.

Bevorzugte Konservierungsmittel umfassen beispielsweise Thiomersal, Organische Quecksilberverbindungen, Phenylquecksilber, Benzalkoniumchlorid, quartäre Ammoniumverbindungen, quartäre Ammoniumsalze, Chlorhexidin, Benzylalkohol, oder Mischungen daraus. Wenn Konservierungsmittel vorhanden sind, liegen diese bevorzugt im Bereich von 0,0005 bis 5 Gew.-% bezogen auf das Gesamtgewicht der Zusammensetzung vor; mehr bevorzugt im Bereich von 0,005 bis 1 Gew-% bezogen auf das Gesamtgewicht der Zusammensetzung. Konservierungsmittel sind vorteilhaft, da sie die Haltbarkeit der Zusammensetzung erhöhen. Sie können allerdings auch nachteilig sein, da sie bei empfindlichen Menschen zu Unverträglichkeiten und/oder allergischen Reaktionen im Auge führen können.

Die Zusammensetzung kann Enzyme umfassen. Bevorzugte Enzyme umfassen Lysozym und/oder Katalase. Am meisten bevorzugt ist Katalase. Wenn Enzyme enthalten sind, umfasst die Zusammensetzung bevorzugt zwischen 100 und 300 Enzymeinheiten U/ml des Enzyms, besonders bevorzugt zwischen 120 und 200 U/ml des Enzyms. Eine Enzymeinheit U entspricht einem µmol Substratumsatz pro Minute. Ein Katal entspricht der Menge an Enzym, die 1 Mol Substrat pro Sekunde (mol/s) umsetzt. Ein U entspricht somit 1/60 mikrokatal (µcat).

Vorzugsweise wird die Osmolarität der Zusammensetzung nicht oder nur minimal durch den Zusatz von Verdickungsmitteln oder weiteren Hilfsstoffen beeinträchtigt.

Vorzugsweise liegt die Zusammensetzung in Form einer wässrigen Lösung, einer Emulsion, einer Suspension, einer Salbe oder eines Gels vor. Vorzugsweise ist die Zusammensetzung zur Applikation als wässrige Lösung, Emulsion, Suspension, Salbe oder Gel hergerichtet. Die Art und Weise der Herrichtung der genannten Applikationsformen ist dem Fachmann bekannt.

Die Sterilisierung der Zusammensetzung erfolgt nach dem Fachmann bekannten Verfahren und Methoden, wie beispielsweise Autoklavieren oder Sterilfiltration.

### Art der Behandlung/Anwendung

Der Begriff *"Symptom einer mit COVID-19 assoziierten sekundären Erkrankung"* umfasst gemäß der vorliegenden Erfindung folgende Symptome, die mit einer COVID-19-Erkrankung einhergehen: Fieber, trockener Husten, Müdigkeit, Gliederschmerzen, Halsschmerzen, Heiserkeit, Durchfall, Kopfschmerzen, Verlust des Geschmacks- oder Geruchssinns, Verfärbungen an Fingern oder Zehen, Hautausschlag, Atembeschwerden oder Kurzatmigkeit, Schmerzen oder Druckgefühl im Brustbereich, Verlust der Sprach- oder Bewegungsfähigkeit, Muskelschmerzen, Rückenschmerzen, Brustschmerzen im Sinne einer Pleuritis, Verminderung der Anzahl der gesamten weißen Blutzellen, Verminderung der Lymphozyten-Anzahl, einer Erhöhung laborchemischer Entzündungsparameter (wie CRP und BSG), Schnupfen, Übelkeit und einer Lungenentzündung (Pneumonie).

Besonders bevorzugt dient die Zusammensetzung zur Anwendung in einem Verfahren für die Behandlung und Prävention von Geruchs- und/oder Geschmacksstörungen, Fieber, Husten, Halsschmerzen, Schnupfen (laufende Nase) oder Mischformen daraus.

Der Begriff "Prävention oder Behandlung von mindestens einem Symptom" meint hierbei, dass mindestens eines der zuvor genannten Symptome abgemildert, gestoppt oder verhindert wird.

Eine Prävention oder Behandlung von nicht mit COVID-19 assoziierten sekundären Erkrankungen, also Erkrankungen, bei denen nicht eine COVID-19-Erkrankung vorangegangen ist oder gleichzeitig stattfindet und/oder kein SARS-CoV-2-Infektionsgeschehen in der Population stattfindet, ist nicht teil der Erfindung.

Die Population wird dadurch bestimmt, dass bei ihr mittels eines Rachen- oder Nasenabstriches in einer Polymerase-Kettenreaktion (PCR), bevorzugt einer quantitativen Echtzeit-Polymerase-Kettenreaktion (RT-PCR), eine SARS-CoV-2-Infektion nachgewiesen wurde. Eine besonders bevorzugte Testmethode zum Nachweis einer SARS-CoV-2-Infektion mittels Echtzeit-Polymerase-Kettenreaktion (RT-PCR) ist beschrieben in der Publikation von Corman et al., Eurosurveillance, veröffentlicht am 23. Januar, 2020.

In weiteren Ausführungsformen kann der Nachweis auch mittels Darmabstrich und PCR-Test erfolgen.

In einem bevorzugten Verfahren wird die Zusammensetzung während der Wachphase des Menschen und mehrmals mit einem zeitlichen Abstand von höchstens 2 h, bevorzugt mit einem zeitlichen Abstand von höchstens 1,5 h, bevorzugt mit einem zeitlichen Abstand von höchstens 1 h, besonders bevorzugt mit einem zeitlichen Abstand von höchstens 0,5 h in das Auge eingebracht.

Somit wird während einer Wachphase von 16 Stunden die Zusammensetzung mindestens 8 Mal, bevorzugt mindestens 10 bis 11 Mal, besonders bevorzugt mindestens 16 Mal, besonders bevorzugt mindestens 32 Mal eingebracht. Bei kürzeren Wachphasen verringert sich die Anzahl der Applikationen entsprechend bzw. bei längeren Wachphasen wird auch die Zahl der Applikationen erhöht.

In einer weiteren Ausführungsform sind auch konsekutive Steigerungen des zeitlichen Abstands zwischen zwei Applikationen möglich. Ein mögliches Verabreichungsschema sieht demnach beispielhaft wie folgt aus: Während der Wachstunden von 12 bis 20 Stunden werden die Augentropfen zunächst in einem Abstand von einer halben Stunde appliziert. Diese Zeitspanne kann bei guter Wirkung dann auf eine Verabreichung pro Stunde oder alle zwei Stunden ausgedehnt werden.

Allerdings sollte der Abstand zwischen zwei Anwendungen in der Regel nicht mehr als 2 Stunden betragen, da ansonsten die positive Wirkung der Behandlung nicht mehr zum Tragen kommt. Daher eignen sich auch im Stande der Technik beschriebene übliche Behandlungsintervalle mit Augentropfen von 2-3 Behandlungen pro Tag bzw. eine Behandlung alle 3 bis 6 Stunden nicht für den erfindungsgemäßen Behandlungseffekt gegen Symptome einer mit COVID-19 assoziierten sekundären Erkrankung.

Die Anwendung der Augentropfen sollte dabei nicht zu früh abgesetzt werden und idealerweise während der gesamten SARS-CoV-2-Infektion des Patienten bzw., insbesondere im Präventiv-Fall, während des gesamten SARS-CoV-2-Infektionsgeschehens in der Population fortgeführt werden.

Unter "Einbringen der Zusammensetzung in das Auge" wird gemäß der vorliegenden Erfindung jede dem Fachmann bekannte Art der Applikation in das Auge verstanden. Darunter fallen beispielsweise Eintröpfeln in das Auge, Eintropfen in den Bindehautsack, Auswaschen des Auges, Spülen des Auges, Besprühen des Auges, Eincremen der Hornhaut, Aufbringen von Gelen oder Salben auf die Hornhaut und weitere bekannte Applikationsarten.

Vorzugsweise wird ein Volumen von 15 bis 250 µl oder ein bis fünf Tropfen der Zusammensetzung innerhalb eines Zeitintervalls, insbesondere pro Anwendung, in das Auge eingebracht. Besonders bevorzugt wird ein Volumen von 30 bis 150 µl der Zusammensetzung oder ein bis drei Tropfen der Zusammensetzung innerhalb eines Zeitintervalls in das Auge eingebracht.

In einem bevorzugten Verfahren wird die Zusammensetzung bereits beim ersten Auftreten von Symptomen einer mit COVID-19 assoziierten sekundären Erkrankung COVID-19, wie Geruchs- und/oder Geschmacksstörungen, Fieber (ab 38,5°C und bis zu 40,4°C Körpertemperatur bei Erwachsenen, in weiteren Ausführungsformen mehr als 40,5°C Körpertemperatur bei Erwachsenen), Husten, Halsschmerzen, Schnupfen, oder Mischformen daraus, in das Auge eingebracht.

In einer weiteren Ausführungsform wird die Zusammensetzung bereits beim ersten Auftreten von unbestimmteren Symptomen wie Halskratzen, Frösteln, erhöhter Temperatur (bis 38,4°C Körpertemperatur bei Erwachsenen), allgemeines Unwohlsein, Kopf- oder Gliederschmerzen, Müdigkeit, Niesen, verstopfter Nase und/oder einer triefenden Nase in das Auge eingebracht, insbesondere wenn diese Symptome während eines SARS-CoV-2-Infektionsgeschehens auftreten.

Eine solche frühzeitige Applikation kann vorteilhafterweise den Krankheitsverlauf mit einer sekundären Infektion verhindern, verkürzen oder stoppen und somit zu einer schnellen Heilung beitragen.

Vorzugsweise wird als Beginn der Applikation ein Zeitpunkt vor dem eigentlichen Ausbruch der Erkältung gewählt. Eine frühzeitige oder gar präventive Applikation ist vorteilhaft, da sie zu einer schnellen Verbesserung der Symptome führen kann. Vorteilhafterweise senkt dies die Wahrscheinlichkeit einer Folgeerkrankung, wie beispielsweise einer Lungenentzündung.

Die präventive Applikation wird vorteilhafter Weise begonnen sobald ein CODIV-19-Infektionsgeschehen in der Bevölkerung auftritt und so lange fortgesetzt, bis das Infektionsgeschehen als beendet gilt.

In diesem Zeitraum wird die Zusammensetzung wenigstens halbstündlich, wenigstens stündlich, wenigstens alle 1,5 Stunden oder wenigstens alle 2 Stunden während der Wachstunden des Patienten in das Auge eingebracht. Vorzugsweise wird die Zusammensetzung in beide Augen eingebracht.

Zum Einbringen in das Auge kann die Zusammensetzung vorzugsweise in Form von Augentropfen, in Form einer Augenspülung, eines Augensprays, einer Augensalbe oder eines Augengels hergerichtet sein. Dies ermöglicht eine einfache Applikation.

Die vorliegende Erfindung bezieht sich auch auf die Behandlung von Patienten mit COVID-19 mit den oben genannten Zusammensetzungen. Das Patientenkollektiv sind Menschen, insbesondere gehört das Patientenkollektiv einer der genannten Populationen an.

Menschen, die in der RT-PCR positiv auf SARS-CoV-2 getestet wurden gehören zum Patientenkollektiv.

Ebenso bezieht sich die vorliegende Erfindung auch auf die Verwendung der beschriebenen Zusammensetzung zur Behandlung und Prävention von mindestens einem Symptom einer mit COVID-19 assoziierten sekundären Erkrankung beim Menschen umfassend das Einbringen der Zusammensetzung in das Auge des Menschen.

### Beispiele

### Beispiel 1

Bei erstem Auftreten von Halsschmerzen während der Corona-Pandemie 2020 in Deutschland mit einem durchschnittlichen Infektionsgeschehen von mehr als 100 Neuinfektionen pro 100.000 Personen, wird dem Patienten die folgende sterile Zusammensetzung in beide Augen eingetröpfelt:
Zusammensetzung I: hypotonische Lösung von NaCl und KCI (275 mOsm/I), 0,24 % Verdickungsmittel, Phosphatpuffer (Di-Natriumphosphat-Dodecahydrat/Na-Dihydrogenphosphat), pH-Wert 7,2.

Der Kanal zwischen Nasenraum und Auge ist bei dem Patienten zu Beginn der Behandlung nicht verstopft.

Die Anwendung erfolgt während der Wachphase von 15h in einem Zeitintervall von 1,5h, zehnmal täglich. Es werden jeweils drei Tropfen der Zusammensetzung in jedes Auge eingetröpfelt.

Bereits am nächsten Tag sind die Halsschmerzen des Patienten abgeklungen. Der Patient ist nach ungefähr 5 Tagen komplett symptomfrei.

### Beispiel 2

Zusammensetzung II: wie Zusammensetzung I, nur zusätzlich 0,5 Gew.-% Na₂EDTA enthaltend.

Bei erstem Auftreten einer verstopften Nase während der Corona-Pandemie 2020 in Deutschland mit einem durchschnittlichen Infektionsgeschehen von mehr als 50 Neuinfektionen pro 100.000 Personen wird dem Patienten die sterile Zusammensetzung II in beide Augen eingetröpfelt.

Die Anwendung erfolgt während der Wachphase von 16h in einem Zeitintervall von 2h, also achtmal täglich. Es werden jeweils drei Tropfen der Zusammensetzung in jedes Auge eingetröpfelt.

Bereits am nächsten Tag ist die Nase des Patienten wieder frei. Der Patient ist nach ungefähr 6 Tagen komplett symptomfrei.

### Beispiel 3

Bei erstem Auftreten von Symptomen während der Corona-Pandemie 2020 in Deutschland unterzieht sich ein Patient einer PCR-Untersuchung (Rachenabstrich) auf SARS-CoV-2 und wird positiv getestet. Der Patient klagt über Halskratzen, leichtes Fieber und trockenen Husten.

Dem Patienten wird die folgende sterile Zusammensetzung in beide Augen eingetröpfelt:
Zusammensetzung I: hypotonische Lösung von NaCl und KCI (275 mOsm/I), 0,24 % Verdickungsmittel, Phosphatpuffer (Di-Natriumphosphat-Dodecahydrat/Na-Dihydrogenphosphat), pH-Wert 7,2.

Die Anwendung erfolgt zunächst an Tag 1 während der Wachphase von 16h zunächst für 6 Anwendungen alle 0,5h, danach dreizehn Mal für 1h. Ab Tag 2 erfolgt die Behandlung während der Wachphasen von ca. 14-16h alle 2 Stunden (ca. 8 Anwendungen/Tag). Es werden jeweils drei Tropfen der Zusammensetzung in jedes Auge eingetröpfelt.

Bereits am zweiten Tag ist das Halskratzen des Patienten abgeklungen. Nach ca. 5 Tagen ist der trockene Husten verschwunden. Der Patient bleibt in den nächsten 14 Tagen komplett symptomfrei.

## Patentansprüche

1. Sterile, wässrige Zusammensetzung zur Anwendung in der Behandlung oder Prävention von mindestens einem Symptom einer mit COVID-19 assoziierten sekundären Erkrankung bei einem Menschen, ausgewählt aus Fieber, trockenem Husten, Müdigkeit, Gliederschmerzen, Halsschmerzen, Durchfall, Kopfschmerzen, Verlust des Geschmacks- oder Geruchssinns, Verfärbungen an Fingern oder Zehen, Hautausschlag, Atembeschwerden oder Kurzatmigkeit, Schmerzen oder Druckgefühl im Brustbereich, Verlust der Sprach- oder Bewegungsfähigkeit, Muskelschmerzen, Rückenschmerzen, Brustschmerzen im Sinne einer Pleuritis, Verminderung der Anzahl der gesamten weißen Blutzellen, Verminderung der Lymphozyten-Anzahl, einer Erhöhung laborchemischer Entzündungsparameter (wie CRP und BSG), Schnupfen, Übelkeit und einer Lungenentzündung (Pneumonie),
wobei der Mensch dadurch definiert wird, dass bei ihm eine SARS-CoV-2-Infektion mittels quantitativer Polymerase-Kettenreaktion (PCR) nachgewiesen wurde, umfassend das Einbringen der Zusammensetzung in das Auge,
wobei die Zusammensetzung während der Wachphase des Menschen und mehrmals mit einem zeitlichen Abstand von höchstens 2 h in das Auge eingebracht wird; wobei die Zusammensetzung eine sterile, wässrige Zusammensetzung einer hypotonischen Lösung von NaCl und KCI (275 mOsm/l) ist, weiterhin umfassend 0,24 % Verdickungsmittel, Phosphatpuffer (Di-Natriumphosphat-Dodecahydrat/Na-Dihydrogenphosphat), pH-Wert 7,2, und optional zusätzlich 0,5 Gew.-% Na₂EDTA,
wobei die Zusammensetzung frei von pharmazeutischen Wirkstoffen, insbesondere frei von abschwellenden Wirkstoffen, Antihistaminika, Antibiotika, Ephedrin und/oder Pseudoephedrin ist.

2. Die Zusammensetzung zur Anwendung gemäß Anspruch 1, wobei der Mensch dadurch definiert wird, dass er sich in einer Population befindet in der die Zahl der nachgewiesenen SARS-CoV-2-Infektionen bei mehr als 100 pro 100.000 Einwohner liegt.

3. Die Zusammensetzung zur Anwendung gemäß einem der vorangehenden Ansprüche, wobei die Zusammensetzung ein Verdickungsmittel ausgewählt aus Polyethylenglykol, Polyvinylpyrrolidon, Cellulose, Cellulosederivaten, Xanthan, Carbomer, Hyaluronsäure und deren Derivaten, sowie Mischungen daraus, umfasst.

4. Die Zusammensetzung zur Anwendung gemäß einem der vorangehenden Ansprüche, wobei die Zusammensetzung während der Wachphase des Menschen und mehrmals mit einem zeitlichen Abstand von höchstens 1,5 h, bevorzugt mit einem zeitlichen Abstand von höchstens 1 h in das Auge eingebracht wird.

5. Die Zusammensetzung zur Anwendung gemäß einem der vorangehenden Ansprüche, wobei Volumen von 15 bis 250 µl der Zusammensetzung pro Applikation in das Auge eingebracht wird; wobei die Zusammensetzung vorzugsweise in beide Augen eingebracht wird.

6. Die Zusammensetzung zur Anwendung gemäß einem der vorangehenden Ansprüche, wobei die Zusammensetzung beim ersten Auftreten eines Symptoms einer mit COVID-19 assoziierten sekundären Erkrankung, wie Geruchs- und/oder Geschmacksstörungen, Fieber, Husten, Halsschmerzen, Halskratzen, Schnupfen (laufende Nase), Müdigkeit oder Mischformen daraus und/oder bei Kontakt mit einem nachweislich mit SARS-CoV-2-Infizierten in das Auge eingebracht wird.

## Claims

1. A sterile, aqueous composition for use in the treatment or prevention of at least one symptom of a secondary disease associated with COVID-19 in a human, selected from fever, dry cough, fatigue, pain in the limbs, sore throat, diarrhea, headache, loss of sense of taste or smell, discolorations of fingers or toes, skin rash, breathing difficulties or shortness of breath, pain or feeling of pressure in the thoracic region, loss of ability to speak or move, muscular pains, back pain, chest pain associated with pleurisy, reduction in the total white blood cell count, reduction of the number of lymphocytes, an increase of laboratory inflammation parameters (such as CRP and ESR), common cold, nausea and a pulmonary inflammation (pneumonia),
wherein the human is **characterized in that** in him or her a SARS-CoV-2 infection has been detected by means of a quantitative polymerase chain reaction (PCR),
comprising administration ofthe composition into the eye,
wherein the composition is applied into the eye during the waking phase of the human and a multiple timesat intervals of at most 2 h;
wherein the composition is a sterile, aqueous composition of a hypotonic solution of NaCl and KCl (275 mOsm/l), furthermore comprising 0.24 % by weight of a thickener, phosphate buffer (di-sodium phosphate dodecahydrate/Na-dihydrogen phosphate), pH value 7.2, and optionally in addition 0.5 % by weight Na₂EDTA,
wherein the composition is free of active pharmaceutical ingredients, in particular free of active decongestantingredients, antihistamines, antibiotics, ephedrine and/or pseudoephedrine.

2. The composition for use according to claim 1, wherein the human is defined by the fact that he or she is part of a population in which the number of the detected SARS-CoV-2 infections is higher than 100 per 100,000 residents.

3. The composition for use according to one of the preceding claims, wherein the composition comprises a thickener selected from polyethylene glycol, polyvinyl pyrrolidone, cellulose, cellulose derivatives, xanthan, carbomer, hyaluronic acid and the derivates thereof, as well as mixtures thereof.

4. The composition for use according to one of the preceding claims, wherein the composition is applied into the eye during the waking phase of the human and multiple times with a period of time of at most 1.5 h in between, preferably with an interval of at most 1 h.

5. The composition for use according to one of the preceding claims, wherein a volume of 15 to 250 µl of the composition per administration is applied into the eye; wherein the composition is preferably applied into both eyes.

6. The composition for use according to one of the preceding claims, wherein the composition is applied into the eye, when a symptom of a secondary disease associated with COVID-19, such as smell and/or taste disorders, fever, cough, sore throat, tickle in the throat, common cold (runny nose), fatigue or combinationsthereof, occurs for the first time and/or in the case of a contact with a person being proven to be infectedwith SARS-CoV-2.

## Revendications

1. Composition aqueuse stérile pour son utilisation dans le traitement ou la prévention d'au moins un symptôme d'une affection secondaire associée à la COVID-19 chez l'être humain, choisi parmi la fièvre, la toux sèche, la fatigue, les douleurs articulaires, les maux de gorge, la diarrhée, les maux de tête, la perte du goût ou de l'odorat, la décoloration des doigts ou des orteils, l'éruption cutanée, les difficultés respiratoires ou l'essoufflement, la douleur ou la sensation de pression dans la zone thoracique, la perte de la capacité de parler ou de se mouvoir, les douleurs musculaires, les maux de dos, les douleurs thoraciques de type pleurésie, la diminution du nombre total de globules blancs, la diminution du nombre de lymphocytes, une augmentation des paramètres inflammatoires de laboratoire (tels que la CRP et la VS), la rhinite, les nausées et une inflammation des poumons (pneumonie),
l'être humain étant défini par le fait qu'une infection par le SRAS-CoV-2 a été détectée chez lui par réaction en chaîne par polymérase (PCR) quantitative,
comprenant l'administration de la composition dans l'œil, laquelle composition est introduite dans l'œil pendant la phase d'éveil de l'être humain et à plusieurs reprises avec un intervalle de temps d'au plus 2 h ;
la composition étant une composition aqueuse stérile d'une solution hypotonique de NaCl et de KCI (275 mOsm/I), comprenant en outre 0,24 % d'épaississant, un tampon phosphate (phosphate disodique dodécahydraté/dihydrogénophosphate de sodium), valeur de pH 7,2, et optionnellement en plus 0,5 % en poids de Na₂EDTA, la composition étant exempte de principes actifs pharmaceutiques, en particulier exempte de principes actifs décongestionnants, d'antihistaminiques, d'antibiotiques, d'éphédrine et/ou de pseudoéphédrine

2. Composition pour son utilisation selon la revendication 1, dans laquelle l'être humain est défini comme appartenant à une population au sein de laquelle le nombre d'infections par le SRAS-CoV-2 détectées est supérieur à 100 pour 100 000 habitants.

3. Composition pour son utilisation selon l'une quelconque des revendications précédentes, dans laquelle la composition comprend un épaississant choisi parmi le polyéthylène glycol, la polyvinylpyrrolidone, la cellulose, les dérivés de cellulose, le xanthane, le carbomère, l'acide hyaluronique et leurs dérivés, ainsi que des mélanges de ceux-ci.

4. Composition pour son utilisation selon l'une quelconque des revendications précédentes, dans laquelle la composition est administrée dans l'œil pendant la phase d'éveil de l'être humain et à plusieurs reprises avec un intervalle de temps d'au plus 1,5 h, de préférence avec un intervalle de temps d'au plus 1 h.

5. Composition pour son utilisation selon l'une quelconque des revendications précédentes, dans laquelle un volume de 15 à 250 µl de la composition est administré dans l'œil par application ; la composition étant de préférence introduite dans les deux yeux.

6. Composition pour son utilisation selon l'une quelconque des revendications précédentes, dans laquelle la composition est administrée dans l'œil dès la première apparition d'un symptôme d'une maladie secondaire associée à la COVID-19, tel qu'une altération de l'odorat et/ou du goût, de la fièvre, de la toux, des maux de gorge, des picotements dans la gorge, une rhinite (écoulement nasal), de la fatigue ou des formes mixtes de ceux-ci et/ou lors d'un contact avec une personne dont l'infection par le SRAS-CoV-2 a été prouvée.
